# EUROPEAN PATENT APPLICATION

(11) **EP 1 095 627 A1**
(43) Date of publication of application: **02.05.2001**
(21) Application number: 00123573.8
(22) Date of filing: 27.10.2000
(51) Int. Cl.: A61B 18/14

(54) **Electrosurgical probe for surface treatment**

(30) Priority: 27.10.1999 US 428229
(71) Applicant: Everest Medical Corporation, Minneapolis, Minnesota 55441 (US)
(72) Inventor: Latterell, Scott T., Minneapolis, Minnesota 55405 (US)
(74) Representative: Kirschner, Klaus Dieter, Dipl.-Phys.

(57) **Abstract**

A surgical tissue treatment device. The device includes an elongated wand housing. The housing carries, proximate its distal end, a first unitary electrode. The first electrode is defined by a wall having an irregular surface formed from a plurality of interwoven electrically-conductive strands such as a metal mesh. A plurality of nodes are formed at interstices of intersections of the strands with one another. In a bipolar embodiment of the invention, a first, or active, electrode cooperates with a second, return electrode which is spaced along an axis of elongation of the wand housing. A high frequency voltage generator is provided to generate a voltage across the first and second electrodes.

## Description

### Technical Field

The present invention deals broadly with instruments used in electrosurgical procedures. More narrowly, however, it deals with instruments used in diminishing, reducing or shrinking organic tissue. A primary focus of the invention is treatment of bodily tissue and, particularly, a device which is efficient in effecting orthopedic ablation.

Depending upon the needs of a particular patient being treated, various types of procedures wherein tissue treatment is electrosurgically performed are dictated. Ablation is one type of procedure wherein damaged or extraneous tissue is liquified and disposed of. The condition of the patient does not, however, always dictate treatment as radical as ablation. For example, certain conditions may merely justify a shrinkage process wherein destruction of tissue, if any does occur, is very minimal. Another process which might be prescribed is resurfacing. In this procedure, tissue may be treated to a minimum extent so as to define a desired contour. Resurfacing may be performed so as to minimize or eliminate additional damage to contiguous tissue.

In performing electrosurgical tissue treatment procedures, various types of instruments are employed. Very commonly, surgeons employ devices for ablation procedures which are bipolar. Such devices provide a pair of electrodes, one active and one return in close proximity to one another, between which a current field is generated to affect the surrounding environment, including tissue, blood, etc.

Bipolar instruments are differentiated from monopolar devices. The latter provide only a single electrode at the site to be affected. The patient on whom the surgery is being performed serves as the location for external grounding. Monopolar devices have the disadvantage that they provide the opportunity for stray energy to affect an unintended site. They tend, however, to have as an advantage the fact that they cut better because of the high current density at the active electrode.

Bipolar technologies have a number of advantages. First, lower energy requirements are necessary relative to monopolar devices. This is so because it is not necessary to "drive" a current through the patient's body in order to accomplish the intended task. Safety is also facilitated with bipolar devices because there tends to be only minimal opportunity for stray energy.

Regardless of whether a particular device is monopolar or bipolar, however, a number of desirable characteristics are sought to be achieved. First, the prior art dictates that an instrument used in such electrosurgical procedures be quick-firing. That is, when the surgeon intends to trigger the device to generate a current field for effecting cutting or other forms of treatment, the response should be as instantaneous as possible so that the field is generated at the intended location. If the responsiveness of the instrument is fast, there is less chance that the instrument will move before the field is generated, and less patience on the part of the surgeon is necessary.

This quick-firing characteristic is determined by a number of factors. Research has revealed that these factors include surface area ratio of the electrodes, electrode material, impedance of the tissue to be treated, level of voltage applied, and nature of the environment (that is, for example, whether it is wet or dry).

Further, it is desirable that an instrument of the type discussed herein be capable of generating as uniform a field as possible. When a substantially uniform field is generated, more consistent and controlled treatment will be able to be accomplished. That is, results will, be more predictable and uniform in the area to be affected, only tissue that is intended to be treated will be ablated or otherwise modified by the field, and tissue intended to be maintained in place will be retained.

In the case of ablation, it is also desirable that the current field generated be adequate to effect the intended ablation while providing adequate hemostasis. If the current density is not sufficient, the procedure being performed, in the best case, might merely be unsuccessful.

A final dictate of the prior art is that there be sufficient coverage accomplished by the current field generated. Sufficient size of the field will enable the surgical procedure to be accomplished much more quickly than would otherwise be the case.

United States Patent No. 5,843,019 issued to Eggers et al. on December 1, 1998 and generally illustrates a typical prior art device. The title of the apparatus of that document is "SHAPED ELECTRODES ... FOR ELECTROSURGICAL CUTTING AND ABLATION". Illustrative of the variations discussed in the document is that illustrated in FIG. 8A. The active electrode is a shaped, loop electrode which is intended to generate a high electric field intensity and associated current density between the active electrode and the target site at which ablation is to be performed. Nevertheless, the apparatus illustrated in that document suffers from a number of the shortcomings implicit in view of the desirable characteristics discussed herein.

It is to the dictates of the prior art and the shortcomings of structures proposed as solutions that the present invention is directed. It is an improved apparatus for use in electrosurgical procedures wherein it is intended to reduce or diminish bodily tissue.

The present invention is a device for surgically treating body tissue. It takes the form of both monopolar and bipolar embodiments. In a monopolar embodiment, the device includes a housing and a first, unitary electrode carried by the housing proximate an extremity thereof. The first, unitary electrode includes electrically-conductive material which defines a wall having an irregular surface. The surface, thereby, has a multiplicity of nodes. A voltage generator and a return electrode pad on the body of the patient are provided to generate a high frequency voltage which concentrates at the unitary electrode. The pad enables circuit grounding.

In a bipolar embodiment, a second, return electrode is included at the device's distal end, but in a direction of the proximal end from the first electrode. The second electrode is spaced in close proximity to the first electrode, and the generator generates a high frequency voltage across both electrodes. Electrode size and spacing are such that the voltage concentrates at the unitary electrode.

The invention includes various structural embodiments. In one embodiment, the second electrode is annular and spaced radially outward from the first unitary electrode. In that embodiment, the device includes a dielectric sleeve which is positioned radially between the first unitary electrode and the second electrode. In that embodiment, the first unitary electrode can be recessed axially within the dielectric sleeve.

In another embodiment, the second electrode can be axially spaced from the first unitary electrode in a direction toward a proximal end of the housing. In that embodiment, the electrically-conductive material of which the first unitary electrode is formed can define a tip of the housing which spans, substantially fully, the cross-sectional area thereof.

The tip can be constructed of flexible, interwoven, electrically-conductive strands. With such a construction, the nodes are defined by interstices formed at intersections of the electrically-conductive strands.

The electrically-conductive strands of the first unitary electrode can be strands of a metallic screen. With such a construction, a dielectric rod received within a lumen through the device housing can, at its distal end, engage the metallic screen. The dielectric rod can be displaceable, within the lumen, to various locations oriented substantially parallel to the axis of elongation of the housing. By so displacing the dielectric rod, the contour of the screen can be restructured to different configurations. Typically, a surface of the distal end of the dielectric rod in engagement with the screen would be convex. In a preferred embodiment, the distal end of the rod can be generally spherical.

It will be understood that a conductive rod could also be used in place of a dielectric rod. When such a conductive rod is employed, it could serve as the electrical connector by which the first unitary electrode is connected to a voltage generator.

Another embodiment of the invention employs a first unitary electrode which takes the form of a metallic screen formed as a generally cylindrical cage. The cage can be made flexible, and a support can be utilized to selectively maintain the cage in an erect configuration.

If desired, a lumen can be defined within the housing. In certain embodiments, an electrically-conductive solution can be flooded in a channel or channels defined within the lumen so that the site of the first unitary electrode can be irrigated. Typically, the irrigation solution would be a saline fluid.

The unitary electrode can be made of various materials. Appropriate materials are stainless steel, a conductively-coated polymer, tungsten, platinum, platinum iridium, gold, and an alloy of tungsten and platinum.

The present invention is thus an improved device for treating body tissue during an electrosurgical procedure. More specific features of the invention and advantages obtained in view of those features will become apparent with reference to the DETAILED DESCRIPTION OF THE INVENTION, appended claims, and accompanying drawing figures.
FIG. 1 is a perspective view of an overall system employing a device in accordance with the present invention;
FIG. 2 is a perspective view of a resecting instrument in accordance with the present invention;
FIG. 3 is a side sectional view of the distal end of one embodiment of a device housing in accordance with the present invention;
FIG. 4 is an enlarged sectional view showing the tip of the device of FIG. 3;
FIG. 5 is a sectional view illustrating an embodiment having a rod for adjusting contours of the active electrode of the device;
FIG. 6 is a greatly enlarged view of a portion of a structure similar to that of FIG. 5 but having a conductive rod;
FIG. 7 is a view similar to FIG. 3, showing an alternative embodiment of the invention;
FIG. 8 is a simplified, side elevational illustration of a monopolar device showing a 90° bend;
FIG. 9 is a view similar to FIG. 8 illustrating a malleable housing wherein the device tip can be bent to a desired shape/angle; and
FIG. 10 is a side elevational view, some portions thereof being broken away, illustrating a device having an axially recessed active electrode.

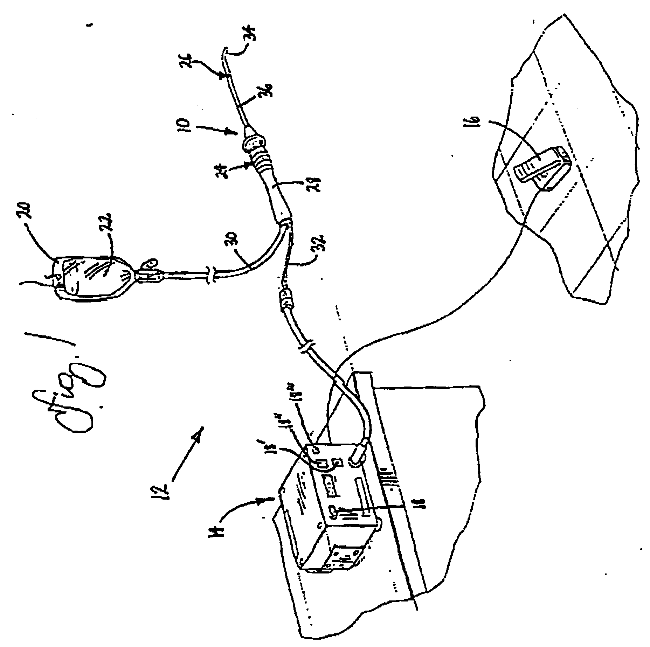

Referring now to the drawings wherein like reference numerals denote like elements throughout the several views, FIG. 1 illustrates a device 10 for electro-surgically treating body tissue in accordance with the present invention, as used in an overall treatment system 12. The overall system 12 includes what is illustrated as a hand-held device 10 which is inserted, typically, through surgical incision in the patient's body. The 10 device is electrically connected to a power supply 14 which generates a high frequency voltage. The voltage extends across, in a bipolar embodiment of the invention, a first, active electrode and a second, return electrode. As will be discussed hereinafter, an electrical field corona is generated proximate the active electrode, the corona serving to treat bodily tissue by shrinking it, eliminating it, etc. The power supply operation can be controlled by means of a foot pedal 16 as illustrated in FIG. 1. Voltage settings and other functions of the power supply 14 can be varied by controls 18, 18', 18'', 18'''on the power supply package.

FIG. 1 also illustrates a source 20 of fluid 22. Such fluid 22 is used to irrigate the area of the target tissue to be treated to improve conductivity between electrodes. The presence of the fluid 22 serves to more effectively generate the electric field corona and maintain and transmit the energy of the field to the target tissue. If desired, a control can be provided on the device for regulating flow of the conductive fluid to the target area.

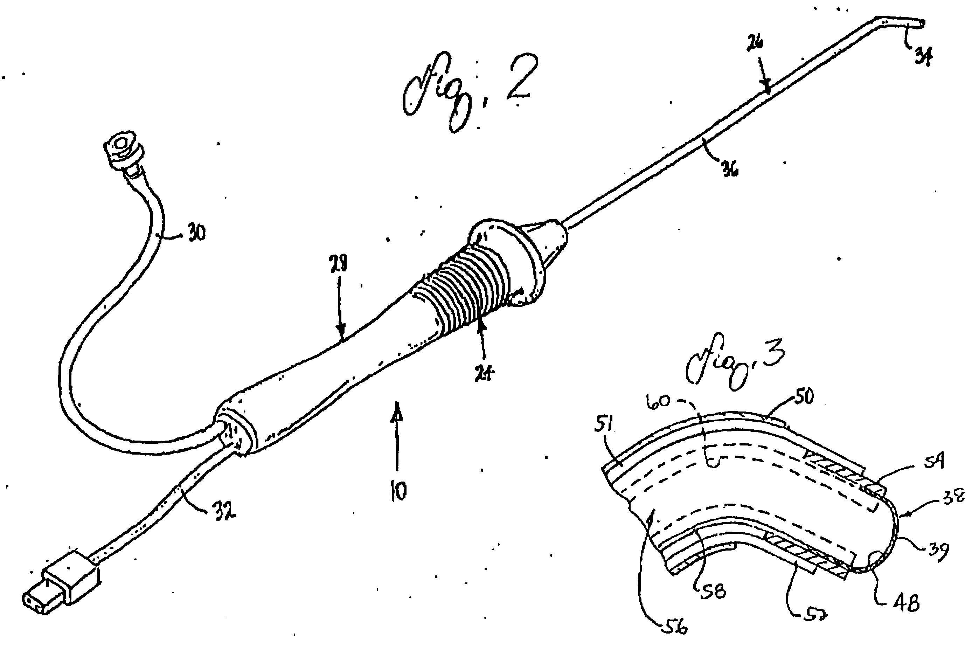

FIG. 2 illustrates in more detail a body tissue treating device in accordance with the present invention. The device comprises a housing 24 which includes a probe or wand portion 26 and a handgrip portion 28. Connective segments 30, 32 are illustrated, the segments serving to enable the device 10 to be attached to the power supply 14 and a source 20 of electrically-conducting fluid 22.

It will be noted that the wand portion 26 of the device 10 is shown as having a portion proximal the handgrip portion 28 which is substantially coaxial with the handgrip portion 28. Additionally, however, the wand portion 26 is shown as including a portion 34 distal from the handgrip 28 which is oriented at an angle with respect to the proximal portion 36 of the wand 26. It has been found that such a relationship enables more facile operation of the device 10, for example, during arthroscopic surgery.

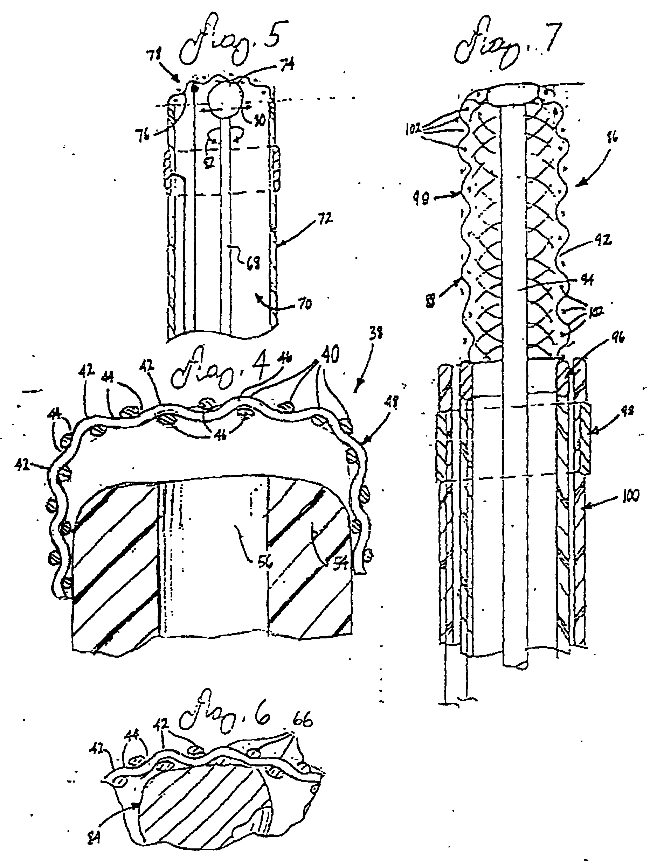

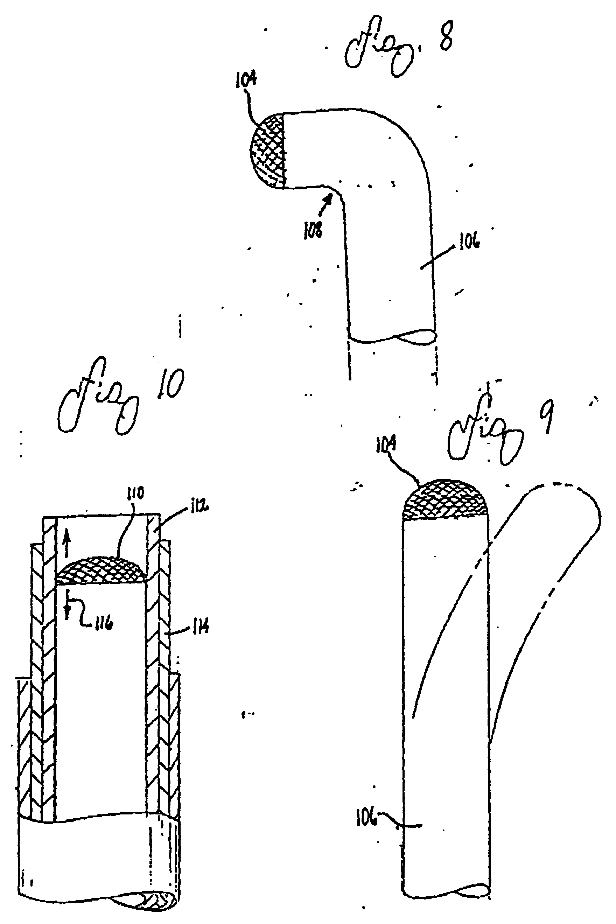

FIG. 3 illustrates a remote end of the distal portion 34 of the wand 26. That figure shows a first unitary electrode 38 which defines the tip of the housing wand portion 26. The first electrode 38, as best seen in FIG. 4, is made to define an irregular surface 39 having a multiplicity of nodes 40. That is, the surface 39 is provided with a multiplicity of convolutions 42 and involutions 44. FIG. 4, which illustrates in more detail a tip of a construction such as that in FIG. 3, shows the tip wherein an electrically-conductive material forming the convolutions 42 and involutions 44 spans, substantially, the full cross-sectional area of the tip.

FIG. 4 illustrates a tip constructed of flexible, interwoven electrically-conductive strands 46. The strands 46, aggregately, form an electrode 38 which has a domed or convex portion. They are interwoven to define a wall 48 which comprises the first, active electrode 38. While not essential to the invention, a preferred embodiment envisions employment of the convex structure illustrated. Such a structure affords better contact with the changing contours of tissue to be treated. Additionally, the convex contour provides both front and side effective zones. The nodes 40 which are formed are defined at interstices of intersections of the electrically-conductive strands 46.

Various materials can be utilized in forming the first electrode 38. Specifically, strands of stainless steel wire, a conductively-coated polymer wire, tungsten wire, platinum wire, gold wire, and an alloy of tungsten and platinum wire are envisioned as being appropriate for this purpose.

FIG. 3 illustrates an insulative layer 50 overlying a substantially rigid conductive tube 51, the exposed end of which comprises return electrode 52. Tube 51 serves as the wand 26, at the end of which the first electrode 38 is mounted. The layer 50, it is intended, is made of an insulative material. It thereby serves to render the outer surface of the wand portion 26 of the housing 24 non-conductive. The second or return electrode 52 is shown as extending axially in a direction beyond the end of the insulative layer 50 and away from the handgrip portion 28 of the housing 24. The conductive tube 51, in FIG. 3, is shown as being tubular, as is the electrode 52, and with the insulative layer 50 substantially conforming to its outer surface.

FIG. 3 also illustrates an insulator tip 54 which is fitted within a lumen 56 defined within the return electrode 52 and at the distal end of the return electrode 52 extending some measure therebeyond. The insulator tip 54 is, typically, made of a ceramic material. It serves to insulate the return electrode 52 from the first, active electrode 38, as will be discussed hereinafter.

FIG. 3 also illustrates the first, active electrode 38 extending from the lumen 56 and held within the distal end of the insulator tip 54. A conductor 58 is shown as electrically connecting the active electrode 38 to a voltage source (not shown in FIG. 3). An electric potential is, thereby, created between the first, active electrode 38 and the second, return electrode 52.

FIG. 3 also illustrates, in phantom, a flexible tube 60 running axially within the lumen 56 defined by the return electrode 52. This tubing 60 is optional and can serve to conduct saline solution, for example, to the site of the active electrode 38. It can also serve to apply suction to the area to remove tissue debris and other material from the site of the active electrode 38.

In FIG. 3, the return electrode 52 is annular in construction. The size and spacing relationship of the active and return electrodes would be made so as to be appropriate in view of various considerations. The axial spacing between the electrodes is a distance appropriate in view of the desired effects, the voltage applied across the electrodes, and other factors. Proper spacing aids in consistent performance, even current density, and lowers risk of carbon tracking between electrodes. Proper spacing, additionally, prevents arcing between electrodes and promotes arcing between the active electrode and tissue.

In order to maximize effectiveness of the device, it is desirable that the ratio of total surface area of the return electrode 52 relative to the active electrode 38 be at least 2:1 and, more desirably, 4:1 or greater. This can be achieved by designing the architecture of the device in a manner wherein the axial dimension of the return electrode 52 is varied and the diameter of the strands of electrically-conducive material of the active electrode 38 and the openness of the weave of those strands is coordinated with the overall surface area of the return electrode 52.

It is desirable to maintain at least this ratio in order to attain the adequate current density in order to achieve desired tissue effect at the active electrode 38, and prevent undesirable current densities at the return electrode 52. Because of the construction of the interwoven active electrode 38, there will tend to be an initial firing at one node 40. In view of the node density and the fact that a multiplicity of nodes 40 exist, the firing will be transmitted over substantially the full surface of the active electrode 38.

Ultimately, the goal is to achieve a balanced design wherein the corona generated at the active electrode 38 is as uniform as possible. It is desirable to have a uniform distribution wherein there is no significant disparity between hot spots and cold spots at the active electrode 38. With a more uniform distribution, a quicker kickoff results.

Another aspect of the balanced design seeks to maintain a substantially uniform distance between the active electrode 38 and the return electrode 52. This aspect also facilitates quick-firing and generation of the corona at the active electrode 38.

As previously, discussed, FIG. 3 illustrates, in order to generate the voltage across the electrodes 38, 52, a conductor 58 leading to the active electrode 38. A conductor 58 extends from the power source 14 to the active electrode 38, and a second conductor (not shown), if necessary, extends from the power source 14 to the conductive tube 51. It will be understood, of course, that the conductive tube 51 may have a relatively long axial dimension. As a result, no appreciably long flaccid conductor need be provided to connect the return electrode 52 to the power source 14. What is important, of course, is that an electrical differential be maintained across the electrodes.

FIG. 5 illustrates an embodiment having a dielectric rod 68 received within a lumen 70 passing through the tubular probe 72. A distal end 74 of the dielectric rod 68 is shown as being in engagement with an underside 76 of the electrically-conductive screen material forming the active electrode 78. FIG. 5 illustrates a series of arrows 80, 82 intended to reflect that the position of the rod 68 can be varied both radially and circumferentially within the lumen 70. By doing so, the configuration of the active electrode 78 can be varied depending upon dictates calling for a particular shape.

FIG. 5 illustrates the dielectric rod 68 as being oriented substantially parallel to the axis of elongation of the probe 72. Further, the surface of the rod 68 in engagement with the active electrode 78 is illustrated as being convex, the distal end of the rod 68 being substantially spherical in form.

FIG. 6 illustrates a variation of the structure shown in FIG. 5. While in FIG. 5 a dielectric rod 68 is employed, FIG. 6 illustrates a rod 84 which not only serves the purpose of the dielectric rod described with reference to FIG. 5, but also functions as the connector to the power supply 14. That is, electrical transmission occurs through the rod 84, in this case conductive, rather than through a separate connector.

FIG. 7 illustrates an additional embodiment encompassed within the scope of the present invention. The embodiment illustrated there includes an active electrode 86 formed as a generally cylindrical cage 88, having, as with the embodiment of FIG. 3 and FIG. 4, a wall 90 having an irregular noded surface 92, which can be flexible in nature. The figure illustrates a support 94 which can be used to selectively maintain the cage 88 in an erect configuration.

FIG. 7 shows a conductive sleeve 96 to connect the active electrode to the power supply 14. In such a ease, the support 94 need not be conductive and would, in fact, be manufactured of a dielectric material. It will be understood, however, that, if ease of manufacture would dictate, the support 94 could be conductive and serve to electrically interconnect the active electrode 86 to the power supply 14. In that configuration of the invention, the sleeve 96, at the distal end of which the active electrode 86 is mounted, would need not be conductive.

In other embodiments, a lower portion of support 94, spaced within active electrode 86, can function as an internal return electrode. With such a configuration, balance and concentricity are more effectively achieved.

FIG. 7 illustrates a return electrode 98 that is generally annular and spaced axially a short distance in a direction toward the handgrip portion 28 of the housing 24 from the active electrode 86. FIG. 7 illustrates the return electrode 98 as being mounted on the outer surface of a dielectric, tubular wand 100. In all embodiments having an annular return electrode as discussed above, it is desirable that the overall surface area of the return electrode be at least 2:1 with respect to the overall surface area of the active electrode.

As illustrated in FIG. 7, the cylindrical cage 88 is defined by a multiplicity of strands 102 of electrical conductive material which are interwoven or braided to define nodes or convolutions. The convolutions cooperate with alternating involutions to thereby form an irregular surface 92.

Firing of the device is, again, rapid and spreads across the active electrode 86. In the case of the embodiment of FIG. 7, however, a corona generated is able to be applied more laterally than is true in the case of the embodiment of FIG. 3.

FIGS. 8 and 9 illustrate monopolar embodiments of the device. In FIG. 8, the active electrode 104 defines the tip of the wand portion 106 of the housing. The wand 106 is, however, permanently bent at a 90° angle. Such an embodiment enables particular modes of operation of the device to be accomplished in view of the bend at 108.

FIG. 9 illustrates an active electrode 104 similar to that of FIG. 8. It is, however, mounted at a distal end of a malleable wand portion 106. In the case of this housing, various configurations of alternative embodiments of monopolar and bipolar devices can be formed. It will be understood that preformed and malleable shafts apply to bipolar designs as well monopolar designs.

In the case of the embodiments of both FIGS. 8 and 9, the body of the patient being treated would serve as ground. Both the benefits and drawbacks of a monopolar structure are inherent in these embodiments.

FIG. 10 illustrates a first unitary electrode 110 which is recessed axially within a dielectric sleeve 112. This particular embodiment, it is envisioned, would employ an annular return electrode 114 insulated from the active electrode 110 by the dielectric sleeve 112. Arrows 116 indicate the embodiment having the active electrode 110 being adjustable.

The embodiment of FIG. 10 enables the corona generated to be more controlled. That is, the portion of the corona generated which extends beyond the distal end of the sleeve 112 can be maintained consistent. It could also be adjusted to obtain the desired results.

An external auxiliary tube (not shown) could be employed. Such an auxiliary tube would be configured with respect to an axis substantially parallel to the axis of the wand portion of the housing. An auxiliary tube of this nature can be employed for various purposes. For example, it could be utilized to channel irrigation and/or flushing fluid to the target site. Alternatively, it could be utilized to introduce vacuum into the target site area to remove abladed tissue from that area. Thus, the auxiliary tube can perform the function of the lumen in FIG. 3.

It will be understood that this disclosure, in many respects, is only illustrative. Changes may be made in details, particularly in matters of shape, size, material, and arrangement of parts without exceeding the scope of the invention. Accordingly, the scope of the invention is as defined in the language of the appended claims.

## Claims

1. A device for surgically treating body tissue, comprising:
(a) an elongated housing;
(b) a first unitary electrode carried by said housing proximate a distal end thereof, said first unitary electrode including electrically-conductive material defining a wall having an irregular surface with a multiplicity of nodes;
(c) a second electrode carried by said housing at a location spaced from said first unitary electrode; and
(d) means for generating a high frequency voltage across said electrodes.

2. A device in accordance with Claim 1 wherein said wall is convex.

3. A device in accordance with Claim 2 wherein said second electrode is annular and spaced radially outward from said first unitary electrode, said device further comprising a dielectric sleeve radially intermediate said electrodes.

4. A device in accordance with Claim 2 wherein said second electrode is spaced from said first unitary electrode in a direction toward a proximal end of said housing.

5. A device in accordance with Claim 2 wherein said wall defines a tip of said housing and spans, substantially fully, a cross-sectional area of said housing.

6. A device in accordance with Claim 5 wherein said wall is constructed of flexible, interwoven, electrically-conductive strands, and wherein said nodes are defined by interstices at intersections of said electrically-conductive strands.

7. A device in accordance with Claim 6 wherein said second electrode is generally annular accommodating a lumen therethrough.

8. A device in accordance with Claim 7 wherein said wall comprises a metallic screen, and wherein said electrodes are spaced along an axis of elongation of said housing, said device further comprising a dielectric rod received within said lumen with a distal end of said dielectric rod in engagement with said metallic screen, said dielectric rod being displaceable to various locations within said lumen to adapt a contour of said metallic screen to desired configurations.

9. A device in accordance with Claim 8 wherein said dielectric rod is oriented substantially parallel to said axis of elongation of said housing.

10. A device in accordance with Claim 8 wherein a surface of said distal end of said dielectric rod in engagement with said metallic screen is convex.

11. A device in accordance with Claim 8 wherein said distal end of said dielectric rod is generally spherical.

12. A device in accordance with Claim 1 wherein said wall defines a generally cylindrical cage.

13. A device in accordance with Claim 12 wherein said generally cylindrical cage is flexible, said device further comprising a support to volitionally maintain said generally cylindrical cage in an erect configuration.

14. A device in accordance with Claim 13 wherein said wall is constructed of flexible, interwoven, electrically-conductive strands, and wherein said nodes are defined by interstices at intersections of said electrically-conductive strands.

15. A device for reducing organic tissue, comprising:
(a) a housing having a distal end, a proximal end, spaced from said distal end along an axis, and a cross-sectional area taken along a plane generally perpendicular to said axis;
(b) a first unitary electrode disposed proximate said distal end of said housing, said first unitary electrode being made of an electrically-conductive material woven from a plurality of conductive strands to define a wall having an irregular surface with a multiplicity of convolutions and involutions;
(c) a second electrode carried by said housing at a location spaced from said first unitary electrode in a direction toward a proximal end of said housing; and
(d) means for generating a high frequency voltage across said electrodes.

16. A device in accordance with Claim 15 wherein said second electrode is generally annular and accommodates a lumen therethrough.

17. A device in accordance with Claim 16 wherein said lumen channels electrically-conductive solution to irrigate said first unitary electrode.

18. An electrosurgical ablation device, comprising:
(a) a wand housing having a distal end and a proximal end spaced from said distal end along an axis of elongation;
(b) a first unitary, electrically-conductive electrode disposed proximate said distal end of said wand housing and made from a plurality of interwoven strands to define a material wall having an irregular surface with a plurality of nodes;
(c) a second electrode carried by said housing at a location spaced from said first unitary electrode; and
(d) means for generating a high frequency voltage across said electrodes.

19. An ablation device in accordance with Claim 18 wherein said wand housing defines, centrally therewithin, a lumen, said first unitary, electrically-conductive electrode being mounted within said lumen.

20. An ablation device in accordance with Claim 19 wherein said second electrode is generally annular and spaced radially outwardly from said first unitary, electrically-conductive electrode, and further comprising an insulative sleeve disposed radially intermediate said first unitary, electrically-conductive electrode and said second electrode.

21. An electrosurgical device for surgically treating body tissue, comprising:
(a) a housing;
(b) a unitary electrode carried by said housing proximate an extremity thereof, said unitary electrode comprising a wall defined by an electrically-conductive material having an irregular surface with a multiplicity of nodes; and
(c) means for generating a voltage at said unitary electrode.

22. A device in accordance with claim 21, wherein said unitary electrode is made of stainless steel, gold, platinum, platinum iridium, tungsten, an alloy of tungsten and platinum or a conductively-coated polymer.

23. A device in accordance with claim 21 further comprising a second electrode carried by said housing at a location spaced from said electrode.

24. A device in accordance with claim 21 further comprising a tube, external to said housing, for evacuating tissue and irrigation fluid from a site being treated.
